Europäisches Patentamt

European Patent Office

Office européen des brevets

(19)

(11) **EP 0 906 969 A1**

## (12) EUROPÄISCHE PATENTANMELDUNG

(43) Veröffentlichungstag:
**07.04.1999 Patentblatt 1999/14**

(51) Int. Cl.⁶: **C23F 11/14**, E21B 41/02,
C07C 233/35

(21) Anmeldenummer: 98118262.9

(22) Anmeldetag: 26.09.1998

(84) Benannte Vertragsstaaten:
**AT BE CH CY DE DK ES FI FR GB GR IE IT LI LU
MC NL PT SE**
Benannte Erstreckungsstaaten:
**AL LT LV MK RO SI**

(30) Priorität: **01.10.1997 US 942444**

(71) Anmelder:
**Degussa Aktiengesellschaft
60311 Frankfurt (DE)**

(72) Erfinder:
**Kissel, Charles L.
CNC Development, Inc.
Anaheim, California 92804 (US)**

(54) **Verfahren und Zusammensetzung zur Inhibierung von Korrosion**

(57)     Die Erfindung lehrt auf C, H, O und N basierende Korrosionsinhibitoren und solche Inhibitoren umfassende Zusammensetzungen, welche bei Temperaturen im Bereich von 150 bis 450 °C wirksam sind. Die Inhibitoren sind Aminoamide der allgemeinen Formel $R^1$-CO-$NR^2$-Z-$NR^3R^4$, wobei Z ein divalenter aliphatischer oder alicyclischer Rest und $R^1$ ein monovalenter Rest mit einem oleophilen Anteil ist. Bevorzugte Stoffe sind Fettsäureamide von Isophorondiamin, 2(3),5(6)-Diaminonorbornylen, 1,8-Diaminomethan und 2,2,4-Trimethyl-1,6-diaminohexan. Die Inhibitoren eignen sich bei Tiefbohrungen nach Gas und Öl, Raffinerievorgängen und geothermischen Tiefbohrungen.

Printed by Xerox (UK) Business Services
2.16.7/3.6

## EP 0 906 969 A1

**Beschreibung**

Die Erfindung betrifft Verfahren zur Korrosionsinhibierung auf Metalloberflächen und ist durch die Verwendung von spezifischen Aminoamiden als Korrosionsinhibitor gekennzeichnet. Dementsprechend werden Zusammensetzungen zur Inhibierung der Korrosion von Metalloberflächen zur Verfügung gestellt. Das Verfahren kann allgemein zur Korrosionsinhibierung auf Metalloberflächen eingesetzt werden, wie beispielsweise im Zusammenhang mit geothermischen Quellen, Raffinerien, Wärmetransportmedien, Schmierstoffflüssigkeiten, Schmierstoffen, Beschichtungsmitteln, Klebstoffen, Flammschutzmitteln, Kunststoffen, Glas und ähnlichem. Im Besonderen ist das Verfahren zur Verhinderung von Korrosion in Verbindung mit Gas- oder Ölquellen geeignet. Ein besonderes Kennzeichen der Erfindung ist die Korrosionsinhibierung bei hohen Temperaturen, wobei sowohl mit erhöhtem als auch ohne erhöhten Druck gearbeitet werden kann.

HINTERGRUND DER ERFINDUNG

Da die Systeme effizienter und komplexer werden, so daß sie auch bei immer ungünstigeren Bedingungen, wie beispielsweise hohen Temperaturen und bei Hochdrucken, eingesetzt werden, besteht ein steigender Bedarf an Korrosionsinhibitoren, die gegenüber hohen Temperaturen unempfindlich sind.

Verschiedene dieser Anlagen benötigen korrosionsinhibierende Chemikalien, die gegenüber hohen Temperaturen beständig sind. Bei verschiedenen Bohrtechniken und beim Fördern von Gas, Erdöl sowie bei tiefen, heißen, geothermischen Quellen können Temperaturen über 150°C, manchmal über 290°C auftreten. Bestimmte Raffinerievorgänge bedürfen zu ihrer Durchführung Korrosionsinhibitoren. Diese müssen beispielsweise 150 bis 350°C widerstehen, um die Korrosion der Ausrüstung zur Gewinnung des Topprodukts zu verhindern. Bei speziellen Crackanlagen und Destillationstürmen sollten die Inhibitoren 200 bis 400°C widerstehen.

Wärmetransportflüssigkeiten, die in Verfahren zur Erwärmung und Kühlung, zur Ventilation und Klimatisierung von Flugzeugen sowie bei stationären Maschinen, wie beispielsweise Gasleitungs-Transmissionskompressoren, eingesetzt werden, benötigen eine thermische Stabilität bis zu 350°C. Diese benötigen Korrosionsinhibitoren, um die Korrosion und die Verschmutzung zu minimieren. Verschiedene Schmierstoffe werden bei hohen Temperaturen eingesetzt, wie beispielsweise in Maschinen und Turbinen, wobei sowohl auf Erdöl basierende als auch synthetische Stoffe verwendet werden. Zahlreiche Beschichtungen, Klebstoffe, härtbare Harze und Flammschutzmittel benötigen ebenfalls eine hohe Temperaturstabilität.

Die interessanten Korrosionsinhibitoren sind filmbildende Materialien. Diese können in geringen Mengen verwendet werden, wobei sie auf verschiedenen Wegen angewandt werden können. Die Korrosionsinhibitoren haben einen lipophilen Rest, der als Barriere gegen Sauerstoff, Wasser und andere korrodierende Stoffe dient, sowie einen polaren Rest, der an die metallischen Oberflächen bindet. Die polaren Reste sollten Amine sein, wobei die Bindung über das freie Elektronenpaar des Stickstoffs und die unbesetzten d-Orbitale des metallischen Molekulargitters gebildet wird. Die lipophilen Gruppen sind gewöhnliche Fettsäureketten mit 4 bis 30 Kohlenstoffatomen. Dementsprechend werden fettähnliche Amine als Korrosionsinhibitoren verwendet.

Von vielen Aminen ist bekannt, daß sie bei hohen Temperaturen instabil sind. Sie können zerfallen, wobei Ammoniak oder andere kleinere Amine, wie beispielsweise Methylamin, sowie Olefine gebildet werden. Diese Olefine können unter den Bedingungen, die zum Abbau führen, weiter reagieren, wobei unlösliche Aromaten und Polymere gebildet werden. Dieser Abbau ist die Ursache dafür, daß die korrosionsinhibierende Wirkung bei hohen Temperaturen abnimmt.

Die Offenlegungsschrift DE-30 29 790 lehrt ein Verfahren zur Korrosionsinhibierung von Metallen in Gasquellen, die hohe Temperaturen und hohe Drücke aufweisen. Bei diesen Verfahren wird ein fettähnliches Amin mit einem hohen Molekulargewicht oder ein N-Alkyl-1,3-propandiamin, bei welchem der Alkylrest 16 bis 30 Kohlenstoffatome enthält, in die Quelle gegeben, wobei das fettähnliche Amin eine Schutzschicht auf der metallischen Ausrüstung bildet.

Es kann gezeigt werden, daß der am meisten bevorzugte Korrosionsinhibitor der obigen Veröffentlichung bei einer Temperatur oberhalb 290°C eine ungenügende Wirksamkeit hat (siehe Beispiel 3 und seine Verwendung). Offensichtlich findet eine signifikante Zersetzung des Korrosionsinhibitors bei solch hohen Temperaturen statt. Ein weiteres Verfahren verwendet eine Kombination der oben erwähnten Amine mit einem Dialkylsulfid - vergl. US-PS 4 350 600. Außer der begrenzten Stabilität der Amine ist eine solche Kombination nachteilig, weil hierbei eine Verbindung eingesetzt wird, die sowohl geruchsintensiv als auch giftig ist.

Gemäß US-PS 3 959 158 kann die Korrosion von Metalloberflächen in Öl- oder Gasquellen, bei hohen Temperaturen, d.h. bei etwa 149 bis 288°C, inhibiert werden, indem in diese Quellen eine korrosionsinhibierende Zusammensetzung eingeführt wird, die ein primäres Fettsäureamin mit 9 bis 24 Kohlenstoffatomen, eine trimerisierte ungesättigte Fettsäure und eine Alkylarylsulfonsäure aufweist, wobei eine Amidbildung verhindert wird. Es wird angenommen, daß die korrosionsinhibierende Wirkung bei Temperaturen oberhalb von 290°C ungenügend ist, wobei diese Temperatur in tiefen Quellen, besonders in Gasquellen auftreten kann.

2

Es ist bekannt, daß an Position 2 substituierte Fettsäuren, wie Nonylphenoxyessigsäure, und Polyamine, wie Alkylendiamine oder davon abgeleitete Imidazoline, oder Isophorondiamin als Korrosionsinhibitor allein verwendet werden können. Gemäß US-PS 3 775 320 führt eine Kombination beider Verbindungen in Form eines Salzes zu einer synergistisch verbesserten korrosionsinhibierenden Wirkung.

Gemäß der Offenlegungsschrift DE-A-26 22 066 kann eine Alkyl- oder Alkenylbernsteinsäure zur Salzbildung mit einem Polyamin verwendet werden. Es wurde vom Erfinder der vorliegenden Anmeldung und von anderen Personen festgestellt, daß die auf 1,2- oder 1,3-Alkylendiaminen oder den entsprechenden Polyalkylen-Polyaminen basierenden Inhibitoren eine begrenzte thermische Stabilität aufweisen. Obwohl diese Verbindungen bei niederen Temperaturen sehr wirksam sind, können sie bei hohen Temperaturen, beispielsweise oberhalb 290°C, nicht eingesetzt werden.

Es wurden Fettsäureamide als Korrosionsinhibitoren verwendet, als die Systeme eine höhere thermische Stabilität benötigten. Diese wurden aus Fettsäuren und Aminen oder fettähnlichen Aminen und Carbonsäuren gebildet. Viele dieser Stoffe sind jedoch in Kohlenwasserstoffen und aromatischen Lösemitteln schlecht löslich, so daß sie schwer eingesetzt werden können.

Wenn Fettsäuren mit einigen Polyamiden behandelt werden, haben die entstehenden Imidazolin- oder Tetrahydropyrimidinstrukturen eine gute korrosionsinhibierende Wirkung bei niederen Temperaturen. Es tritt jedoch ein starker Abbau auf, falls mit diesen Stoffen behandelte Systeme hohen Temperaturen unterliegen. Dieser verursacht eine abnehmende Schutzwirkung, erhöhte Verschmutzung und in einigen Fällen eine verstärkte Korrosion. Des weiteren werden diese Stoffe leicht hydrolysiert.

Gemäß US-PS 3 412 024 kann die Korrosion von Eisen oder Stahlröhren und anderen Materialien, die aus eisenhaltigen Metallen gebildet werden, die mit korrodierenden süßen und sauren Rohölen in Kontakt kommen, vor allem jenen, die korrodierende Laugen enthalten, durch Behandlung dieser Metalle mit einer korrosionsinhibierenden Zusammensetzung vermindert oder minimiert werden. Diese Zusammensetzung enthalten ein Salz von (i) Alkylbenzolsulfonsäuren und partiellen Amiden oder (ii) Monomeren, Dimeren oder Trimeren höherer Fettsäuren. Diese partiellen Amide sind Aminoamide, die aus monomeren, dimeren oder trimeren Fettsäuren und Polyalkylen-Polyaminen mit 3 bis 10 Aminogruppen gebildet werden. Von den bevorzugten auf Diethylentriamin und Dipropylen-1,2-triamin basierenden Amiden ist bekannt, daß sie leicht in weniger stabile Imidazoline umgewandelt werden. Es wurde vom Erfinder dieser Anmeldung festgestellt, daß die thermische Stabilität dieser Amide für die Verwendung als Korrosionsinhibitoren in Öl- und Gasquellen bei hohen Temperaturen, beispielsweise bei 290 bis 340°C, zu gering ist. Ein weiterer Nachteil besteht darin, daß die inhibierende Zusammensetzung eine schwefelhaltigen Verbindung enthält.

Gemäß JP 04/202 396 A (Derwent Abstract 92-29558[36]WPIDS) enthält ein Schmiermittel, welches bei Düsen eine verbesserte Druckverlustrate und Lebensdauer erzeugt, ein carbonsäureamidartiges Wachs, das durch die Umsetzung einer höheren aliphatischen Monocarbonsäure oder deren Mischungen mit einer polybasischen Säure mit einem Diamin, beispielsweise Isophorondiamin, m-Xylylendiamin oder einem $\alpha,\omega$-Alkylendiamin mit 2 bis 6 Kohlenstoffatomen erhalten werden kann. Da das Verhältnis Diamin zu Monocarbonsäure zwischen 0,5 und 2 liegt, umfaßt das Reaktionsprodukt Aminoamide. Es gibt in dieser Japanischen Patentveröffentlichung keine Hinweise auf eine korrosionsinhibierende Wirkung dieser Substanzen. Es wird auch nicht erwähnt, daß bestimmte Aminoamide eine viel höhere thermische Stabilität haben als andere, so daß diese dementsprechend bei höheren Temperaturen eingesetzt werden können. Es wurde durch den Erfinder der vorliegenden Anmeldung festgestellt, daß die thermische Stabilität der meisten erwähnten Aminoamide gering ist. Die einzige Ausnahme sind Aminoamide, die auf eine höhere Fettsäure und Isophorondiamin basieren.

Ein weiteres Aminoamid wird in U.S.-Patent Nr. 5 391 826 offenbart. Es ist dadurch gekennzeichnet, daß es ein Diamidotriamin aus der Umsetzung von Glutaminsäure und Isophorondiamin ist. In dem Dokument wird offenbart, daß das Produkt als Rohmaterial für die Herstellung von Treibstoff und Schmierstoffadditiven eingesetzt werden kann, aber es gibt keine weiteren Hinweise auf deren Herstellung und auf ihre Wirkung.

Es wurden Versuche unternommen, Heteroverbindungen, die auf Schwefel, Phosphor und Silicium basieren, als Korrosionsinhibitoren für hohe Temperaturen zu verwenden. Diese Materialien können jedoch neue Probleme hervorrufen, wie beispielsweise eine Katalysatorvergiftung in Raffinationsanlagen, beschleunigte Korrosion durch Abbauprodukte und Umweltgefährdungen, die mit der erhöhten Freisetzung von Stoffen aus den behandelten Systemen zusammenhängen (beispielsweise Nox, Sox, Phosphate, usw.).

Dementsprechend sind Materialien, die auf Kohlenstoff, Wasserstoff, Sauerstoff und Stickstoff basieren, bevorzugte Kandidaten für hochtemperaturbeständige Korrosionsinhibitoren.

Demgemäß ist es Aufgabe dieser Erfindung einen neuen auf C, H, O und N basierenden Korrosionsinhibitor zu schaffen, welcher selbst bei hohen Temperaturen im wesentlichen wirksam bleibt, d.h. im Bereich von 150°C bis etwa 450°C.

Es ist eine weitere Aufgabe ein Verfahren zur Korrosionsinhibierung in diesem hohen Temperaturbereich zur Verfügung zu stellen, wobei sowohl mit als auch ohne erhöhten Druck gearbeitet werden kann. Das Verfahren und die Zusammensetzungen können dementsprechend bei hohen Temperaturen in Gas- oder Ölquellen sowie in anderen

EP 0 906 969 A1

Umgebungen eingesetzt werden, die hierin beispielhaft offenbart werden.

ZUSAMMENFASSUNG DER ERFINDUNG

Es wurde festgestellt, daß durch die Umsetzung von Carbonsäuren, vorzugsweise Fettsäuren, mit verzweigten oder cyclischen Polyaminen, vorzugsweise Diaminen, welche keine signifikanten Mengen an fünf- oder sechsgliedrigen Ringen bilden, Aminoamide entstehen, die eine ausgezeichnete thermische Stabilität und Löslichkeit sowie eine ausgezeichnete Wirkung als Korrosionsinhibitoren haben.

Im besonderen sind diese Verbindungen für die Verwendung in Bohrflüssigkeiten für tiefe, heiße Gasquellen, Ölquellen und geothermischen Quellen sowie für bestimmte Raffinerievorrichtungen und in anderen Gebieten geeignet. Diese Verbindungen sind ebenfalls als Additiv geeignet, wie beispielsweise für Wärmetransportflüssigkeiten, Schmierflüssigkeiten, Schmiermittel, Beschichtungen, Klebstoffe, Kunststoffe, Flammschutzmittel und ähnliche.

Zur Korrosionsinhibierung auf Metalloberflächen werden diese Aminoamide in flüssiger Phase in einer wirksamen Menge mit diesen Metalloberflächen in Kontakt gebracht, wobei die flüssige Phase der Korrosionsinhibitor als solcher in geschmolzener Form, eine Lösung oder eine Dispersion dieses Inhibitors sein kann.

Der Korrosionsinhibitor ist ein Aminoamid der allgemeinen Formel

$$R^1\text{-CO-NR}^2\text{-Z-NR}^3R^4 \tag{I}$$

oder Mischungen hiervon, worin Z ein divalenter aliphatischer Rest ist, der eine verzweigte Kohlenstoffkette hat, oder ein divalentes alicyclisches Ringsystem einschließlich von Ringsystemen mit ein oder zwei gebundenen Alkylengruppen und/oder Alkylgruppen, wobei die Reste eine Kohlenstoffkette mit wenigstens drei Kohlenstoffatomen zwischen den gebundenen $NR^2$- und $NR^3R^4$-Gruppen aufweisen und worin das Strukturelement $-NR^2\text{-Z-NR}^3R^4$ der Formel (I) kein Imidazolin- oder Tetrahydropyrimidin-Ringsystem bildet.

$R^2$, $R^3$ und $R^4$ bedeuten jeweils unabhängig voneinander Wasserstoff, einen aliphatischen, alicyclischen oder aromatischen Rest, wobei aromatische Reste weniger bevorzugt sind.

$R^1$ ist ein monovalenter Monomer- oder Polymerrest, welcher einen olephilen Anteil hat, der einen gesättigten oder olefinischen, linearen oder verzweigten Kohlenwasserstoffrest, einen alicyclischen oder aromatischen Rest aufweist, wobei der aromatische Rest weniger bevorzugt und ein Alkyl, Alkenyl oder Alkadienylrest mit 3 bis 30 Kohlenstoffatomen, vorzugsweise mit 7 bis 22 Kohlenstoffatomen, bevorzugt ist sowie ein Salz davon. Die $R^1$-Reste können ebenfalls ein oder mehrere Substituenten ausgewählt aus der Gruppe bestehend aus Carboxyl, Amino und Hydroxyl enthalten. Die „alicyclischen" und „aromatischen" Reste umfassen ebenfalls die alkylsubstituierten Reste.

BEVORZUGTE AUSFÜHRUNGSFORM

Die Aminoamidkorrosionsinhibitoren können auf allgemein bekannte Weise hergestellt werden. Eine Carbonsäure der allgemeinen Formel $R^1$-COOH oder ein Derivat hiervon, wie beispielsweise

ein Ester $R^1\text{-COOR}^5$, worin $R^5$ ein Kohlenwasserstoffrest, wie beispielsweise Alkyl und ähnliche ist,

ein Amid $R1\text{-CONR}^6R^7$, worin $R^6$ und $R^7$ Wasserstoff oder ein Kohlenwasserstoffrest, wie beispielsweise ein Alkyl oder ähnliche sind,

ein Säurehalogenid, $R^1COX$, worin X ein Halogen ist, oder

ein Säureanhydrid, $R^1CO_2R^1$,

wird mit einem Amin der allgemeinen Formel $R^2HN\text{-Z-NR}^3R^4$ umgesetzt, worin $R^1$, $R^2$, $R^3$, $R^4$ und Z die oben genannte Bedeutung haben. Wahrend der Amidierung, die gewöhnlich bei Temperaturen von etwa 100°C bis 300°C, vorzugsweise 150°C bis 250°C über 0,5 bis 20 Stunden stattfindet, wird das Aminoamid (I) gebildet. Bei der Reaktion können Mischungen von Aminoamiden gebildet werden, wobei diese als solche eingesetzt werden, oder gereinigt werden können, um die reine Verbindung zu erhalten.

Die Aminverbindung der Formel $R^2HN\text{-Z-NR}^3R^4$ ist vorzugsweise ein Diamin, aber ein oder mehrere der Gruppen $R^2$, $R^3$ und $R^4$ können weitere Aminogruppen, beispielsweie Aminoalkylgruppen, aufweisen. Die Aminoalkylgruppen der Reste $R^2$, $R^3$ oder $R^4$, welche ein Imidazolin oder Tetrahydropyrimidinring bilden können, sind ausgeschlossen. $R^2$, $R^3$ und $R^4$ sind vorzugsweise Wasserstoff oder eine niedere Alkylgruppe mit 1 bis 5 Kohlenstoffatome, die jeweils gleich oder verschieden sein können. Beispiele sind u.a. Methyl, Ethyl, n-Propyl, Isopropyl, wobei Methyl bevorzugt ist. Am meisten bevorzugt sind Diamine der Formel $H_2N\text{-Z-NH}_2$.

4

Eine besonders interessante Gruppe von Aminen, welche erwähnt werden sollte, sind jene in denen zumindest eines der Kohlenstoffatome β zu der vorhandenen Aminogruppe oder -gruppen quartär ist.

Eine bevorzugte Bedeutung für Z ist eine verzweigte Alkylengruppe, worin die Kette zwischen den Aminogruppen, die an Z gebunden sind, zumindest drei Kohlenstoffatome hat, und diese Kette zumindest einen, vorzugsweise mehr als einen, niederen Alkylsubstituenten, wie Methyl und Ethyl hat. Es ist wichtig, daß Z so aufgebaut ist, daß das Strukturelement $-NR^2-Z-NR^3R^4$ der Formel (I) im wesentlichen kein Imidazolin oder Tetrahydropyrimidin-Ringsystem bildet. Besonders bevorzugte verzweigte Alkylengruppen haben 6 bis 10 Kohlenstoffatome. Beispiele für Diamine der Formel $H_2N-Z-NH_2$ sind: 2,2-Dimethyl-1,3-diaminopropan, 2-Methyl-1,4-diaminobutan, 2-Ethyl-1,5-diaminopentan, 2,4,4- oder 1,1,3-Trimethyl-1,6-diaminohexan, 2-Methyl-1,5-diaminopentan, 2-Ethyl-2-butyl-1,5-diaminopentan. Beispiele für Triamine sind 4-Aminomethyl-1,8-diaminooctan und N-substituiertes Di-1,3-propylentriamin, worin die Substituenten so aufgebaut sind, daß eine Ringbildung vermieden wird. Andere, welche erwähnt werden können, sind 1-(2-Aminomethyl)-piperazin, 1-(2-Aminomethyl)-4-aminopiperidin und 4-Methyl-1,4,7-triazaheptan.

Andere bevorzugte Bedeutungen für Z umfassen ein mono-, di- oder tricyclisches fünf- oder sechs-gliedriges cycloaliphatisches Ringsystem. Bei dieser Bedeutung enthält Z vorzugsweise 6 bis 12 Kohlenstoffatome. Das Ringsystem kann niedere Alkylsubstituenten, beispielsweise Methyl, Ethyl, n- oder iso-Propyl, aufweisen. Ein oder beide Aminogruppen, die an Z gebunden sind, können hieran direkt über ein sekundäres oder tertiäres Kohlenstoffatom des Ringsystems oder über einen Alkylsubstituenten gebunden werden. Beispiele des cyclischen Diamins der Formel $H_2N-Z-NH_2$ sind: 1,4- und 1,3-Diaminocyclohexan (das trans-Isomer der beiden, des 1,4- und 1,3- Diaminocyclohexans, ist bevorzugt, weil dieses Isomer keine Ringe bilden kann. Um die Ringbildung des cis-Isomers zu verhindern, sollte zumindest ein Stickstoffatom des Diamins ausreichend substituiert sein.), 1,3- oder 1,4-Bis(aminomethyl)cyclohexan, Bis(4-aminocyclohexyl)methan, Bis(4-amino-3-methylcyclohexyl)methan, 1-Amino-3-aminomethyl-3,5,5-trimethylcyclohexan(=Isophorondiamin), 3(4), 8(9)-Bis(aminomethyl)-tricyclo$[5,2,1,0^{2.6}]$decan, 2(3), 5(6)-Bis(aminomethyl)-bicyclo[2,2,1]heptan (=Diaminomethyl-norbornylen), 1,8-Diamino-1-methyl-4-isopropyl-cyclohexan(=1,8-Diaminomenthan) und 3-Cyclohexylaminopropylamin.

Z kann ebenfalls ein divalenter aromatischer oder aromatisch-aliphatischer Rest sein, wie beispielsweise meta- oder para-Phenylen oder $-C_6H_4-(CH_2)_3$, aber gewöhnlich sind diese Reste weniger bevorzugt als vergleichbare aliphatische oder alicyclische Reste.

Der $R^1$-Rest des Carbonsäureteils des Aminoamids kann linear oder verzweigt, aliphatisch oder cycloaliphatisch, gesättigt oder ungesättigt oder aromatisch sein, wobei aromatische Reste weniger bevorzugt sind. Der $R^1$-Rest kann ebenfalls ein oder mehrere Substituenten enthalten, die aus der Gruppe bestehend aus Carboxyl, Amino und Hydroxyl ausgewählt sind. Reine Carbonsäure oder Mischungen von natürlichen oder synthetischen Carbonsäuren können verwendet werden. Eine lineare Alkyl oder Aklenylgrupe $R^1$ mit 7 bis 22 Kohlenstoffatomen ist am meisten bevorzugt.

Falls die Gruppen $R^2$, $R^3$ oder $R^4$ olephil genug sind, kann $R^1$ ein kurzkettiger Rest sein, andernfalls ist ein Rest $R^1$ mit mindestens vier Kohlenstoffatomen bevorzugt.

Beispiele gesättigter aliphatischer Fettsäuren sind: Ameisensäure, Essigsäure, Propionsäure, Buttersäure, Valeriansäure, Capronsäure, Heptansäure, Caprylsäure, Nonansäure, Caprinsäure, Undecansäure, Laurinsäure, Tridecansäure, Myricasäure, Pentadecansäure, Palmitinsäure, Heptadecansäure, Stearinsäure, Nonadecansäure, Eicosansäure, Heneicosansäure, Docosansäure, Trocosansäure, Tetracosansäure, Pentacosansäure, Cerotinsäure, Heptacosansäure, Montansäure, Noncosansäure, Melissinsäure und dergl.

Beispiele für ethylenisch ungesättigte aliphatische Carbonsäuren sind die Pentensäuren, die Hexensäure, die Octensäuren, die Nonensäuren, die Decensäuren, die Tridecensäuren, die Tetradecensäuren, die Pentadecensäuren, die Hexadecensäuren, die Heptadecensäuren, die Octadecensäuren, die Nonadecensäuren, die Eisosensäuren, die Decosensäuren, die Tetracosensäuren.

Beispiele der cycloaliphatischen Säuren sind Naphthensäuren, Kohlenwasserstoffsäuren und Chaulmoograsäuren, Cyclopentancarbonsäuren, Cyclohexencarbonsäuren, Camphersäure und Fencholsäure.

Beispiele gemischter Fettsäuren sind jene, die aus Schmalz, Kokosnussöl, Rapssamenöl, Sesamöl, Tallöl, Palmöl, Palmkernöl, Olivenöl, Maisöl, Baumwollsamenöl, Sardinenöl, Talgöl, Sojabohnenöl, Erdnussöl, Rizinusöl, Robbenöl, Schieferöl, Haiöl, anderen Fischölen, Teesamenöl, partiell oder vollständig hydrierte tierische oder pflanzliche Öle oder Öle erhältlich aus Wachsen, wie Bienenwachs, Walrat, Montanwachs, Japanwachs, Coccerin-und Carnubawachs, Paraffinwachs, Rohvaselin, napthenartige und oxidierten Fetten gewonnen werden können.

Die Verwendung von Di- oder Polycarbonsäure, vorzugsweise in Kombination mit Monocarbonsäuren, ist ebenfalls möglich. Beispiele aliphatischer Polycarbonsäuren sind Oxalsäure, Malonsäure, Succinsäure, Glutarsäure, Adipinsäure, Pimelinsäure, Suberinsäure, Azelainsäure, Sebacinsäure, Nonandicarbonsäure, Decandicarbonsäure, Undecandicarbonsäure, Tartarsäure, Fumarsäure, Maleinsäure, Mesocinsäure, Zitraconsäure, Glutonsäure, Itaconsäure, Muconsäure, Aconitsäure, und ähnliche. Großtechnische Polymere, beispielsweise dimere und höhere Fettsäuren sind ebenfalls für diese Erfindung zweckmäßig.

Ein Beispiel einer Hydroxylgruppen enthaltenden Säure der Formel $R^1$-COOH ist 9,10-Dihydroxystearinsäure.

Beispiele einer Aminogruppen enthaltenden Säure sind 6-Aminocapronsäure, Asparaginsäure und Glutaminsäure.

Des weiteren kann das Verhältnis der Fettsäure- zur Aminofunktionalität in einem weiten Bereich liegen. Gewöhnlich beträgt das Verhältnis etwa eine Carbonsäuregruppe pro zwei Aminogruppen in dem Di- oder Polyaminrohmaterial. Andere Verhältnisse können jedoch ebenfalls zweckmäßig sein. Das Verhältnis kann beispielsweise einen Überschuß an Fettsäure oder an Polyamin aufweisen. Als eine weitere Ausführungsform können Salze der Aminoamide ebenfalls geeignet sein. Salze können durch nicht-umgesetzte Fettsäuren und die Aminogruppe des Aminoamidprodukts gebildet werden. Salze können ebenfalls durch Zugabe anderer Säuren zu den Aminoamidprodukten gebildet werden. Die anderen Säuren können Fettsäuren, Hydroxycarbonsäuren, wie beispielsweise Hydroxyessigsäure, Mineralsäuren, wie beispielsweise Salzsäure, polymere Säuren, wie beispielsweise Polyacryl- und Polymethacrylsäuren und ihre copolymeren Polysäuren mit einer Vielzahl von Comonomeren sein. Comonomere, welche erwähnt werden sollten, sind Crotonsäure, Fumarsäure, Itaconsäure, Maleinsäure, Allylalkohol, Acrylamid, Methacrylamid, Acrylnitril, Acrolein, Methacrolein, Butylacrylat, Butylmethacrylat, Ethylacrylat, Ethylmethacrylat, Methylacrylat, Methylmethacrylat, Vinylacetat, 2-Hydroxyethylacrylat, 2-Hydroxyethylmethacrylat, Styrol, Butadien, 2-Carboxyethylacrylat und 2-Carboxyethylmethacrylat.

Für die vorliegende Erfindung sind zusätzlich zu den obigen Beispielen auch Mischungen der einzelnen Komponenten geeignet.

Erfindungsgemäß wird eine Korrosionsinhibierung auf metallischen Oberflächen, wie beispielsweise Weichstahl (unlegierter Stahl, C-Stahl), Schmiedeeisen, Gußeisen, Edelstahl und ähnliche, erreicht, indem diese Metalloberfläche mit einer wirksamen Menge des erfindungsgemäßen Aminoamidkorrosionsinhibitors in Kontakt gebracht wird. Wo fließende Fluidsysteme den Korrosionsinhibitor enthalten, die mit der metallischen Oberfläche in Kontakt treten, beträgt die wirksame Menge etwa 1 bis 100 Teile oder mehr des Aminoamids pro eine Million Teile des Fluids. Bei statischen Fluidsystemen, beispielsweise Chargenverfahren, beträgt die Menge etwa 1 bis 10 000 Teilen pro eine Million Teile des Fluids. Die Inhibierung kann ebenfalls durch Tauchen, Einreiben, Besprühen, usw. der Metalloberfläche mit reinem Aminoamidinhibitor (als solchem) oder einer Mischung erreicht werden, die den Inhibitor, beispielsweise in einer Menge von etwa 1 bis 99 Gew.-% der Zusammensetzung enthält.

Die Erfindung stellt ebenfalls neuartige Aminoamidprodukte der allgemeinen Formel $R^1$-CO-NR$^2$-Z-NR$^3$R$^4$ zur Verfügung. Diese sind Amidierungsprodukte der Amine der allgemeinen Formeln $R^2$-NH-Z-NR$^3$R$^4$ mit $R^1$-COOH oder eines reaktiven Derivats hiervon mit der Bedingung, daß Fettsäureamide des Isophorondiamins und Diamidotriamine, die auf Glutaminsäure basieren, ausgeschlossen sind. Unter Berücksichtigung der zuvor genannten Ausnahmen sind $R^1$, $R^2$, $R^3$, $R^4$ und Z wie hierin zuvor definiert. Besonders interessant sind Aminoamide, die auf einer Fettsäure mit 4 bis 30 und mehr Kohlenstoffatomen, vorzugsweise mit 8 bis 18 Kohlenstoffatomen, welche gesättigt sein kann oder olefinische Gruppen aufweisen kann, sowie einem verzweigten oder cyclischen Diamin, vorzugsweise einem der folgenden Amine basieren: 2,2-Dimethyl-1,3-diaminopropan, 2-Methyl-1,4-diaminobutan, 2-Ethyl-1,5-diaminpentan, 2,2,4- oder 1,1,3-Trimethyl-1,6-diaminohexan, 2-Methyl-1,5-diaminopentan und 2-Ethyl-2-butyl-1,5-diaminopentan, trans-1,4- und trans-1,3-Diaminocyclohexan, 1,3- oder 1,4-Bis (aminomethyl)cyclohexan, Bis(4-aminocyclohexyl)methan, Bis(4-amino-3-methylcyclohexyl)methan, 1-Amino-3-aminomethyl-3,5,5-trimethylcyclohexan (=Isophorondiamin), 3(4), 8(9)-Bis(aminomethyl)-tricyclo[5,2,1,0$^{2.6}$]decan, 2(3), 5(6)-Bis(aminomethyl)-bicyclo[2,2,1]heptan (=Diaminomethyl-norbornylen) und 1,8-Diamino-1-methyl-4-isopropyl-cyclohexan(=1,8-Diaminomenthan).

In dem erfindungsgemäßen Verfahren werden die obigen Aminoamide als Korrosionsinhibitoren in roher Form oder als Teil einer korrosionsinhibierenden Zusammensetzung verwendet, welche Lösungen mit verschiedenen Lösemitteln umfaßt. Zu den geeigneten Lösemittel gehören unter anderem aliphatische, cycloaliphatische und aromatische Lösemittel, synthetische Alkylbenzole und Kohlenwasserstoffpolymere.

Beispiele der aliphatischen Lösemittel sind Hexane, Heptane, Octane, Nonane, Decane, Undecane, Dodecane, Tridecane, Tetradecane, Pentadecane, Hexadecane, Petrolether, Benzine, Diesel, Düsentreibstoffe, Kerosine, Terpentine, verzweigte Aliphaten, wie beispielsweise Isooctan, Cycloaliphaten, wie beispielsweise Cyclohexan und Methylcyclohexan und Decahydronaphthaline. Mischungen der Kohlenwasserstofföle, Wachse und Fette sind erfindungsgemäß ebenfalls geeignet.

Beispiele aromatischer Lösemittel sind: Benzol, Toluol, Xylole, Mesitylen, Dihydronaphthalin, Tetrahydronaphthalin, Cymol, Cumol, Ethylbenzol, Propylbenzol, Butylbenzol, Phenylpentane, Phenylhexane, Phenylheptane, Phenyloctane, Phenylnonane, Phenyldecane, Phenylundecane, Phenyldodecane, Phenyltridecane, Phenyltetradecane, Phenylpentadecane, Phenylhexadecane, Phenylheptadecane, Phenyloctadecane und ähnliche. Mischungen aromatischer Lösungsmittel, wie beispielsweise schwere aromatische Lösungsmittel, sowie Mischungen der obigen sind erfindungsgemäß ebenfalls geeignet.

Beispiele polymerer Kohlenwasserstofflösungsmittel sind: Polybutadiene, Polybutene, Polypropylene und Mischungen hiervon.

In den meisten Fällen ist der geeignete Korrosionsinhibitor in Öl löslich. In einigen Fällen ist es jedoch vorteilhaft, daß der Inhibitor in Wasser dispergiert wird. Dies kann beispielsweise durch die Verwendung der oben erwähnten Salzderivate geschehen. Dies kann ebenfalls durch die Verwendung von Colösemitteln erreicht werden. Beispiele geeigneter Colösemittel sind: Methanol, Ethanol, Isopropanol, Butanole, Ethylhexanol, Methylpropanole, Carbitole, Cellosolve, Dimethylformamid, Methylpyrrolidon, Dimethylsulfoxid, Glyme, Diglyme, Ethylenglycol, Diethylenglycol und ähnliche. Einige dieser Stoffe fungieren ebenfalls als Frostschutzmittel, beispielsweise Ethylenglycol.

Zur Zusammensetzung zur Inhibierung von Korrosion können Zusatzstoffe beigemischt werden, insbesondere Tenside. Diese Tenside können anionisch, kationisch oder neutral sein. Beispiele sind Alkylsulf(on)ate, Alkylcarboxylate, quartäre Ammoniumsalze sowie Polyoxyethylene, Polyoxypropylene, Polyoxybutylene und verschiedene Mischungen der zuvor genannten Stoffe.

Im allgemeinen weist die Zusammensetzung zur Inhibierung der Korrosion die Aminoamide der obigen allgemeinen Formel (I) oder Mischungen hiervon auf, wobei diese in einem aliphatischen, aromatischen, alkylaromatischen oder cycloaliphatischen Kohlenwasserstofflosungsmittel oder einem Kohlenwasserstoffpolymer gelöst werden oder in Wasser oder einer wässrig-organischen Lösungsmittelmischung dispergiert werden. Die Menge des Aminoamids oder der Mischung hiervon liegt im Bereich von 1 bis 99 Gew.-% der Zusammensetzung. Die Menge liegt vorzugsweise im Bereich von 1 bis 60 Gew.-% und besonders bevorzugt im Bereich von 10 bis 50 Gew.-%.

Regelmäßig findet die Korrosion als allgemeine Korrosion statt, obwohl auch andere spezielle Arten existieren. Geeignete Korrosionsinhibierungsmechanismen können auf der Neutralisation von korrodierenden Mitteln und/oder auf der Bildung eines Schutzfilms auf der Metalloberfläche beruhen. Steigende Temperaturen erhöhen im allgemeinen die Korrosionsgeschwindigkeit. Ein erhöhter Druck kann ebenfalls die Korrosion beschleunigen, weil korrodierende Mittel, wie $CO_2$ und $H_2S$, in einer flüssigen Phase bis zu einer hochkorrodierenden Konzentration gelöst werden können. Durch die Verwendung der erfindungsgemäßen Aminoamide kann die korrodierende Wirkung von $CO_2$, $H_2S$, Laugen (Salzlaugen), Sauerstoff und Luft vermindert werden. Diese Aminoamide können ebenfalls die Korrosion reduzieren, die durch Säuren verursacht wird.

Aminoamidkorrosionsinhibitoren dieser Erfindung neutralisieren korrodierende Säuren mit ihrer Aminogruppe. Die Aminogruppe ermöglicht ebenfalls das Binden an die Metalloberfläche, wobei ein Schutzfilm gebildet wird. Die Inhibitoren sind dadurch gekennzeichnet, daß sie eine unerwartet hohe thermische Stabilität haben, und deshalb auch bei Temperaturen eingesetzt werden können, die größer als 150°C und sogar größer als 190°C sind. Es wurde festgestellt, daß die korrosionsinhibierende Wirkung auch bei sehr hohen Temperaturen im Bereich von 290°C bis 400°C und selbst bei höheren Temperaturen, wie beispielsweise bei 450°C, erreicht wird.

Korrosionsschutz kann dementsprechend durch Verwendung der erfindungsgemäßen Aminoamide mit guter Wirksamkeit selbst in jenen Gebieten erreicht werden, bei denen andere Inhibitoren versagen. Die Aminoamide können als solche oder durch kontinuierliches Beimischen in eine Flüssigkeit an oder oberhalb der Fläche eingesetzt werden, die behandelt werden soll. Alternativ können die Aminoamide halbkontinuierlich oder chargenweise nach gewünschten Intervallen angewendet werden. Bei bestimmten Systemen zur Ölförderung kann die Chemikalie über eine pressartige Vorgehensweise eingesetzt werden. In diesem Fall wird das System rückgeflutet und die Chemikalie wird unterhalb der zu behandelnden Fläche eingesetzt. Der Rückfluß verursacht, daß die Chemikalie die Zielzone passiert und über diese Zone hinweg geleitet wird. Das System wird anschließend in die normale Richtung geleitet und die Chemikalie durchquert die Zielzone nochmals. Es ist natürlich wichtig, daß die Chemikalie den oder die Punkte des Systems erreicht, an denen eine korrodierende Wirkung auftritt, wobei dieses auf verschiedene Arten erreicht werden kann, wobei diese in der Fachwelt bekannt sind. In fließenden Systemen können „stingers" verwendet werden, wobei die Chemikalie in den fließenden Strom injiziert wird, wie beispielsweise in ein Vorratsgefäß oder eine Leitung. Die Aminoamide können ebenfalls Schmiermittelzusammensetzungen beigegeben werden, welche einfach auf das zu schützende Metall gegeben werden.

Wenn die erfindungsgemäßen Aminoamide eingesetzt werden, und das System hohen Temperaturen oder sporadischen Hochtemperatur/Niedertemperaturzyklen ausgesetzt wird, wird durch diese Stoffe Korrosionsschutz erreicht, wenn normale Diamine, Imidazoline und Tetrahydropyrimidine oder Salze hiervon infolge von Abbau versagen. Des weiteren haben diese Aminoamide eine bessere Löslichkeit als einige der gegenwärtigen Inhibitoren. Die verbesserte thermische Stabilität dieser Aminoamide bedeutet des weiteren, daß eine geringere Verschmutzung beobachtet wird, wenn diese eingesetzt werden. Dementsprechend ist es nicht notwendig, die Stahlteile häufig zu ersetzen.

Zu den Gebieten, die von den Vorteilen durch die Anwendung der erfindungsgemäßen Aminoamide profitieren, gehören unter anderem heiße Tiefbohrungen nach Gas und Öl sowie nach Quellen, um Erdwärme zu gewinnen, wobei Bodentemperaturen oberhalb 150°C oder selbst oberhalb 190°C auftreten, und Raffinerievorgänge mit Systemtemperaturen bis zu oder selbst oberhalb etwa 400°C, vor allem wo mögliche Katalysatorgifte durch Heteroatome enthaltende Stoffe ein ernsthaftes Problem darstellen. Beschichtungen zum Schutz von Metallen bei hohen Temperaturen können ebenfalls von der Zugabe der Aminoamidkorrosionsinhibitoren dieser Erfindung als Zusatzstoffe für deren Beschichtungsformulierungen profitieren.

Die Verwendung dieser Aminoamide in Schmierstoffen, Wärmetransportflüssigkeiten, sowie als Additive für Kunststoffe von metallischen Oberflächen gegen Korrision sind weitere Anwendungen.

Hinsichtlich Schmierstoffen und Flüssigkeiten zur Metallbearbeitung stellen die US-PS 4 210 542, 4 196 094, 4 259 206 und 4 273 664 eine ausführliche Abhandlung zur Verfügung. Durch Ersatz von den beschriebenen Organostickstoffverbindungen dieser Patente mit den erfindungsgemäßen Aminoamiden können unter Bedingungen, die den beschriebenen ähnlich sind, höhere thermische Stabilitäten und damit besseren Korrosionsschutz erreicht werden.

Das US-PS 4 261 842 beschreibt die Verwendung von Aminen als Korrosionsinhibitor in der Glasverarbeitung. Die Korrosionsinhibitoren werden in wässerigem Medium verwendet. Falls die Organostickstoffverbindung, die in diesem Patent offenbart werden, durch die erfindungsgemäßen Amide als Salz oder in Colösemittel eingesetzt werden, zeigen diese eine überlegene Wirksamkeit.

Verfahren und Vorrichtungen zur Korrosionsinhibitoren in Raffinerien, Erdölquellen und bei Bohrungen sind in der Fachwelt weithin bekannt, wobei die erfindungsgemäßen Aminoamidkorrosionsinhibitoren durch Austausch der zuvor verwendeten Inhibitoren eingesetzt werden können. Beispielhaft wird auf das Dokument „Corrosion Inhibitors", herausgegeben von C.C. Nathan, National Association of Corrosion Engineers, Houston, Texas (1973) und im besonderen auf die Seiten 46-47, 65-68 und 81 sowie 106 und 108-111 Bezug genommen.

In Verbindung mit dem Einsatz der Aminoamide in Kunststoffen wird bemerkt, daß die meisten Kunststoffe aus Monomeren über Katalysatoren hergestellt werden. Die meisten Katalysatoren sind sauer, wobei sie im fertigen Kunststoffmaterial verbleiben. Wenn diese Kunststoffe hohen Temperaturen ausgesetzt werden, werden die sauren Stoffe freigesetzt, wobei sie die Korrosion von verschiedenen Metalloberflächen verursachen. Einige dieser korrodierenden Mittel, die durch den Abbau des Kunststoff freigesetzt werden, sind: Salzsäure, Essigsäure, Ameisensäure, Formaldehyd, $SO_x$, $NO_x$, $PO_x$ und $H_x$. Die Ausrüstung, die zum Formen, Extrudieren, Verformen usw. eingesetzt wird, kann durch den abgebauten Kunststoff korrodiert werden. Die erfindungsgemäßen Inhibitoren können in den Kunststoff bei der Herstellung zugegeben werden, so daß sie bei ihrer weiteren Verarbeitung eine verminderte korrodierte Wirkung haben.

Kunststoffe werden häufig in verschiedenen Anwendungen eingesetzt, wobei sie hohen Temperaturen ausgesetzt werden. Diese umfassen Anlagen, die aus Kunststoffen hergestellt werden, wie z.B. Kessel, Reaktorleitungen, Vorratsgefäße, Mischer, Ventile, usw., sowie Beschichtungen, Klebstoffe, Dichtungen, Abdichtmittel, und Isolatoren für Ausstattungen. Durch Zugabe der erfindungsgemäßen Aminoamidinhibitoren zu den Kunststoffen wird die Korrosion von Metallen, die mit diesen Kunststoff in Kontakt kommen, verhindert.

Kunststoffe, die hierbei erwähnt werden können, sind: ABS (ACS, ASA), Acetalhomopolymere, Acetalcopolymere, Polyvinylchloride, Acrylharzderivate, Bismaleinimide, Cellulosederivate, Epoxiderivate, Fluorkunststoffe, Ionomere, Flüssigkristallkunststoffe, Melamine, Nitrilharze, Nylonkunststoffe, Phenolharze, Phenylenharze, Polyamidpolyimide, Polyacrylate, Polyarylsulfone, Polymethacrylharzderivate, Polybutylene, Polycarbonate, Polyester, Thermoplasten, Duroplasten, Polyetheretherketone, Polyetherimide, Polyethylene, Polymethylpentene, Polystyrole, Polysulfone, Polyurethane, Polyvinyle, Vinylharzderivate, Polysilicone, Styrolcopolymere, Polyurethane, Vinylidenchloridcopolymere u.a., sowie Mischungen der zuvor genannten.

Eine gleiche Problematik betriff Elastomere. Dies sind natürliche Kautschuke, Latices, Ethylen/Acrylharzderivate, Polythioether, Ethylen/-Propylencopolymere, epoxidierte natürliche Kautschuke, Polyphenylensulfide, Polyamid/Polyimide, Fluorelastomere, Silikonkautschuke, phosphonitrilartige Fluorelastomere, Nitrilelastomere, Epichlornitrilelastomere. Die Aminoamide können auf gleiche Weise wie in Kunststoffe inkorporiert werden. Ein weiteres mögliches Einsatzgebiet der Aminoamide besteht in der Beigabe zu Zement (Beton) sowie zu Keramiken, welche mit Metallen eingesetzt werden. Durch Zugabe dieser Inhibitoren kann die Korrosion von Metall, das den Zement berührt, verhindert werden.

Die Erfindung wird durch die folgenden Beispiele näher erläutert aber nicht begrenzt.

BEISPIEL 1

Herstellung von Imidazolin aus Talgölfettsäure und Diethylentriamin:

In einen Vierhalsrundkolben wurden 251,12 g Talgölfettsäure (Acintol FA-2, Arizona Chemical Company) und 92,53 g Diethylentriamin (Union Carbide) gegeben. Der Rundkolben wurde mit einem Magnetrührer, einem Thermometer und einem Abscheider mit Rückflußkühler ausgestattet. Der Rundkolben wurde unter Stickstoff gehalten und 12 Stunden auf 250°C erwärmt. Für den Erwärmungsprozess wurde eine Stufe Technik mit den folgenden Temperatur/Zeit-Daten verwendet: 105°C/50 Min., 157°C/115 Min., 205°C/6 Std., 245°C/10 Std., 250°C/12 Std.. Während der Heizperiode wurden 35 ml Kondensat in dem Abscheider gebildet, worin 29 ml Wasser, etwa 1 g Ausgangstriamin und 6 ml ölige Schicht aus Triamin und niedermolekulargewichtigen Carbonsäureresten enthalten waren. Die Stufentechnik verminderte die Schaumbildung während der Umsetzung in dem Gefäß. Die Umsetzung führte zu 260 g des Imidazolinprodukts. Dieses Imidazolin war in Hexadecan, einem synthetischen aromatischen Lösungsmittel (Alkylat 230, Monsanto) und p-Cymol löslich, wobei es einen Fließpunkt von -32°C (ASTM D97-66) und IR-Banden (roh) bei 3265 (breit), 2915, 2845, 1605, 1460, 1255, 1013, 988 und 725 $cm^{-1}$ aufwies.

Die Fettsäurezusammensetzung des eingesetzten Actinol FA-2 ist wie folgt:

| | |
|---|---|
| Palmitinsäure | 0,1% |
| Palmitolsäure | 0,2% |
| unbekannte Säure | 0,1% |
| Sterinsäure | 2,5% |
| Ölsäure | 49,5% |
| unbekannte Säure | 1,6% |
| Linolensäure (cis-9, cis-12) | 35,7% |
| unbekannte Säure | 3,1% |
| unbekannte Säure | 0,4% |
| Linolensäure (cis-9, trans-11) | 2,6% |
| Eicosansäure | 1,4% |
| Linolensäure (trans-9, trans-11) | 1,2% |
| Eicosansäure | 0,5% |
| Eicosatriensäure | 0,4% |
| Behensäure | 0,7%. |

BEISPIEL 2

Herstellung von Imidazolin aus Talgölfettsäure und Hydroxyethylethylendiamin:

In einen 500 ml Vierhalsrundkolben wurden 250,00 g Talgölfettsäure (Acintol FA-2, Arizona Chemical Company) und 98,42 g N-Hydroxyethylethylendiamin gegeben. Die Umsetzung wurde wie in Beispiel 1 beschrieben durchgeführt. Nach 12 Stunden wurde die Reaktion angehalten. Die Temperatur innerhalb des Reaktionsgefäßes hatte 248°C erreicht und der Abscheider enthielt 39 ml wässerige Phase und 8 ml Ölschicht. Durch die Umsetzung wurden 302 g Imidazolinprodukt erhalten. Das erhaltene Imidazolin war nicht in Hexadecan aber in einem synthetischen aromatischen Lösungsmittel (Alkylat 230, Monsanto) und p-Cymol löslich, wobei es einen Schmelzpunkt von 32-38°C sowie IR-Banden (roh) bei 3290, 2920, 2825, 1630, 1555, 1540, 1460, 1415, 1265 und 720 cm$^{-1}$ aufwies.

BEISPIEL 3

Herstellung von N-Oleyl-1,3-Propandiamin aus Oleylamin und 3-Chlor-1-aminopropan

In einen 500 ml Dreihalskolben wurden 267,5 g Oleylamin und 98,25 g 3-Chlor-1-aminopropan gegeben. Der Rundkolben wurde mit Magnetrührer, Thermometer und Rückflußkühler ausgestattet. Die Mischung wurde 20 Stunden auf 100°C erhitzt und anschließend auf Raumtemperatur gekühlt. Die rohe Mischung wurde in einen Scheidetrichter gegeben, wonach 150 g Wasser, welches 100 g Natriumhydroxid enthielt, langsam zugegeben wurde. Zusätzliche Natriumhydroxidstücke wurden zugegeben, bis keines mehr gelöst werden konnte. Die Mischung wurde geschüttelt, wobei zwei Schichten gebildet wurden. Die obere Ölphase wurde entfernt und in 250 ml getrocknetes Tetrahydrofuran gegeben. Die Lösung wurde mit wasserfreiem Natriumcarbonat getrocknet, wonach das Tetrahydrofuran bei reduziertem Druck entfernt wurde. Auf diese Weise wurden 227 g Oleyldiamin erhalten. Dieses fettähnliche Diamin hatte einen Schmelzpunkt bei 24 bis 29°C und war nicht in Hexadecan, einem synthetischen aromatischen Lösungsmittel (Alkylat 230, Monsanto) oder p-Cymol löslich, wobei es IR-Banden (roh) bei 3210 (breit), 2920, 2850, 1605, 1460, 1375, 1130, 975 und 725 cm$^{-1}$ aufwies.

Beispiel 4

Herstellung von Aminoamid aus Talgölfettsäure und Isophorondiamin

In einen 100 ml Dreihalsrundkolben wurden 33,6 g Talgölfettsäure (Acintol FA-2, Arizona Chemical Company) und 20,44 g Isophorondiamin (Degamin IPDA, Degussa AG) gegeben. Der Rundkolben wurde mit Magnetrührer, Thermometer und einem Abscheider mit Kühler ausgestattet. Die Reaktionsmischung wurde eine Stunde bei einer gestaffelten Temperatur von 180°C bis 306°C erwärmt. Etwa 0,1 g nicht umgesetztes Isophorondiamin wurde im Kondensat festgestellt. Die Ausbeute dieser Umsetzung betrug 50,8 g des Aminoamidprodukts. Das Aminoamid hatte einen Fließpunkt bei -21°C (ASTM D97-66) und war in Hexadecan, in einem synthetischen aromatischen Lösungsmittel (Alkylat 230, Monsanto) und p-Cymol löslich, wobei es IR-Banden (roh) bei 3290, 3060, 2965, 2850, 1640, 1545, 1460, 1385, 1365, 1305, 1250, 1220, 970 und 750 cm$^{-1}$ aufwies.

BEISPIEL 5

Darstellung von Aminoamid aus Talgölfettsäure und 1,8-Diamino-para-menthan

In einen 100 ml Dreihalsrundkolben wurden 24,12 g Talgölfettsäure (Acintol FA-2, Arizona Chemical Company) und 23,84 g 1,8-Diamino-para-menthan (Aldrich Chemical Company) gegeben. Der Rundkolben wurde mit Magnetrührer, Thermometer und einem Abscheider mit Kühler ausgestattet. Die Mischung wurde 85 Minuten bis zu einer Temperatur von 298°C erwärmt, anschließend auf Raumtemperatur gekühlt. Der Abscheider enthielt 2,5 ml Wasser mit 0,1 g des Ausgangsdiamins sowie 0,6 ml eines öligen Stoffes. Die Ausbeute dieses Verfahrens betrug 44,44 g des Aminoamidprodukts. Das Aminoamidprodukt war nicht in Hexadecan, aber in einem synthetischen aromatischen Lösungsmittel (Alkylat 230, Monsanto) und p-Cymol löslich, wobei es IR-Banden (roh) bei 3280, 3050, 2910, 2840, 1630, 1535, 1445, 1360, 1270, 1173, 1135, 970, 812 und 722 cm$^{-1}$ aufwies.

BEISPIEL 6

Herstellung von Aminoamid aus Talgölfettsäure und m-Xylylendiamin

In einen Dreihalsrundkolben wurden 53,83 g Talgölfettsäure (Acintol FA-2, Arizona Chemical Company) und 25,88 g m-Xylendiamin (Aldrich chemical Company) gegeben. Der Rundkolben wurde mit Magnetrührer, Thermometer und Abscheider mit Kühler ausgestattet. Die Mischung wurde 1 Stunde erwärmt, wobei im Gefäß eine Temperatur von 266°C erreicht wurde. Die Mischung wurde gekühlt. Der Abscheider enthielt 3,3 ml Wasser mit 0,1 g nicht umgesetztem Diamin. Die Ausbeute dieser Umsetzung betrug 75,07 g des Aminoamidprodukts. Dieses Aminoamidprodukt hatte einen Schmelzpunkt von 34 bis 43°C und war nicht in Hexadecan, aber in einem synthetischen aromatischen Lösungsmittel (Alkylat 230, Monsanto) und p-Cymol löslich, wobei es IR-Banden (roh) bei 3190, 3050, 3010, 2900 (breit), 1655, 1635, 1610, 1560, 1545, 1525, 1465, 1440, 1425, 1380, 1350, 1330, 1250, 1160, 1030, 895, 795 und 705 cm$^{-1}$ aufwies.

BEISPIEL 7

Herstellung von Aminoamid aus Talgölfettsäuren und 1,4-Phenylendiamin

In einen 100 ml Dreihalsrundkolben wurden 36,14 g Talgölfettsäure (Acintol FA-2, Arizona Chemical Company) und 14,06 g 1,4-Phenylendiamin (Aldrich Chemical Company) gegeben. Der Rundkolben wurde mit Magnetrührer, Thermometer und Abscheider mit Kühler ausgestattet. Die Mischung wurde eine Stunde erwärmt, bis im Gefäß eine Temperatur von 282°C erreicht wurde. Die Mischung wurde gekühlt. Der Abscheider enthielt, 2,2 ml Wasser mit 0,1 g nicht umgesetztem Diamin. Die Ausbeute dieser 47,69 g des Aminoamidprodukts. Dieses Aminoamid hatte einen Schmelzpunkt von 84 bis 91°C und war nicht in Hexadecan, einem synthetischen aromatischen Lösungsmittel (Alkylat 230, Monsanto) oder p-Cymol löslich, wobei es IR-Banden (roh) bei 3360, 3345, 3290, 3000, 2910, 2840, 1645, 1590, 1520 (breit), 1455, 1420, 1395, 1370, 1300, 1255, 1180, 1110, 1090, 960, 830, 710 (breit) und 505 cm$^{-1}$ aufwies.

BEISPIEL 8

Herstellung von Aminoamid aus Talgölfettsäure und 1,8-Diaminooctan

In einen 100 ml Rundkolben wurden 38,92 g Talgölfettsäure (Acintol FA-2, Arizona Chemical Company) und 20,2 g 1,8-Diaminooctan (Aldrich Chemical Company) gegeben. Der Rundkolben wurde mit Magnetrührer, Thermometer und Abscheider mit Kühler ausgestattet. Die Mischung wurde 34 Minuten erwärmt, wobei im Gefäß 246°C erreicht wurden. Die Mischung wurde anschließend gekühlt. Der Abscheider enthielt, 2,5 ml Wasser mit 0,1 nicht umgesetztem Diamin. Die Ausbeute dieser Umsetzung betrug 51,75 g des Aminoamidprodukts. Das Aminoamid hatte einen Schmelzpunkt von 54 bis 70°C und war nicht in Hexadecan, einem synthetischen aromatischen Lösungsmittel (Alkylat 230, Monsanto) oder p-Cymol löslich, wobei es IR-Banden (roh) bei 3210, 3050, 3010, 2800 (breit), 1630, 1545, 1525, 1460, 1418, 1375, 1260, 1228, 1194, 1076, 945, 820, 725 (s, breit) und 565 cm$^{-1}$ aufwies.

BEISPIEL 9

Herstellung von Aminoamid aus Talgölfettsäure und Diaminomethylnorbornylen

In einen 100 ml Dreihalsrundkolben wurden 33,36 g Talgölfettsäure (Acintol FA-2, Arizona Chemical Company) und 18,51 g Diaminomethylnorbornylen (NBDA, Mitsui Toaksu Chemicals, Inc.) gegeben. Der Kolben wurde mit Magnetrührer, Thermometer und Abscheider mit Kühler ausgestattet. Die Mischung wurde 45 Minuten erwärmt, wobei im Gefäß eine Temperatur von 306°C erreicht wurde. Danach wurde die Mischung gekühlt. Der Abscheider enthielt 2,0 ml Wasser mit 0,1 g nicht-umgesetztem Diamin. Dieses Verfahren lieferte 48,88 g Aminoamid. Das Aminoamid war nicht in Hexadecan, aber in einem synthetischen aromatischen Lösungsmittel (Alkylat 230, Monsanto) und p-Cymol löslich, wobei es einen Fließpunkt von -17°C (ASTM D97-66) und IR-Banden (roh) bei 3280, 3065, 3005, 2920, 2850, 1630, 1545, 1455, 1378, 1260 und 725 cm$^{-1}$ aufwies.

BEISPIEL 10

Herstellung von Aminoamid aus Talgölfettsäure und 2,2,4-Trimethyl-1,6-Hexandiamin

In einen 100 ml Dreihalsrundkolben wurden 33,36 g Talgölfettsäure (Acintol FA-2, Arizona Chemical Company) und 19,0 g 2,2,4-Trimethyl-1,6-hexandiamin (Vestamin TMD, Huls) gegeben. Der Rundkolben wurde mit Magnetrührer, Thermometer und Abscheider mit Kühler ausgestattet. Die Mischung wurde 33 Minuten erwärmt, wobei im Gefäß 268°C erreicht wurden. Die Mischung wurde anschließend gekühlt. Der Abscheider enthielt 2,1 ml Wasser mit 0,1 g nicht umgesetztem Diamin. Die Ausbeute dieser Umsetzung betrug 49,26 g des Aminoamidprodukt. Das Aminoamid hatte einen Fließpunkt von -22°C (ASTM D97-66), und war in Hexadecan, einem synthetischen aromatischen Lösungsmittel (Alkylat 230, Monsanto) und p-Cymol löslich, wobei es IR-Banden (roh) bei 3275, 3025, 2997, 2915, 2845, 1635, 1460, 1365, 1255 und 725 cm$^{-1}$ aufwies.

BEISPIEL 11

Thermische Abbauversuche bei 290°C unter Luft mit 1010 Weichstahl

In 1 ml Glasampullen wurden 25 ml der jeweiligen Probe und 4 cm 1010 Weichstahldraht der Stärke 22 gegeben. Die Ampullen wurden unter Luft verschweißt und anschließend in einen Muffelofen gegeben, der eine konstante Temperatur von 290°C aufwies. Danach wurden die Ampullen aus dem Ofen genommen, gekühlt und anschließend geöffnet. Der Inhalt wurde einer Infrarotanalyse unterzogen. Das Verhältnis der Absorption der Frequenz der aktiven Chemikalie gegen die einer inaktiven Kohenwasserstoff-Frequenz wurde bestimmt und diese wurden anschließend auf das Verhältnis für unbehandelten Fall normalisiert. Tabelle 1 zeigt die verwendeten Infrarotfrequenzen für jede untersuchte Chemikalie und Tabelle 2 stellt die normalisierten Daten gegen die Zeit dar.

Tabelle 1

| Infrarotfrequenzen der untersuchten Chemikalien | | |
|---|---|---|
| Beispiel der untersuchten Chemikalie | Aktive Frequenz (cm$^{-1}$) | Inaktive Frequenz (cm$^{1}$) |
| 1 | 1605 | 1460 |
| 2 | 1630 | 1460 |
| 3 | 1130 | 1460 |
| 4 | 1640 | 1460 |
| 5 | 1630 | 1445 |

Tabelle 1 (fortgesetzt)

| Infrarotfrequenzen der untersuchten Chemikalien | | |
|---|---|---|
| Beispiel der untersuchten Chemikalie | Aktive Frequenz (cm$^{-1}$) | Inaktive Frequenz (cm$^{1}$) |
| 6 | 1635 | 1465 |
| 7 | 1645 | 1515 |
| 8 | 1630 | 1460 |
| 9 | 1630 | 1455 |
| 10 | 1635 | 1460 |

Alle in Tabelle 1 dargestellten Infrarotfrequenzen wurden durch die Analyse der rohen Probe in einem Beckmann Acculab 6 Doppelstrahlspektrometer unter Verwendung von Kaliumbromidsalzplatten erhalten. Die aktiven Frequenzen der Beispiele 1 und 2 sind die Imidazolinbanden, bei Beispiel 3 war es die Bande des sekundären Amins, bei allen anderen war es die Aminoamidcarbonylbande.

Tabelle 2

| Ergebnisse des Abbaus von verschiedenen Chemikalien bei 290°C unter Luft mit 1010 Weichstahl | | | | | |
|---|---|---|---|---|---|
| Normalisiertes Verhältnis (aktiv/inaktiv) | | | | | |
| Beispiel Nr. | nach 10 Std. | nach 20 Std. | nach 30 Std. | nach 40 Std. | nach 50 Std. |
| 1 | 0,98 | 0,97 | 0,96 | 0,955 | 0,95 |
| 2 | 0,95 | 0,895 | 0,865 | 0,845 | 0,83 |
| 3 | 0,51 | 0,38 | 0,3 | - | - |
| 4 | 0,99 | 0,98 | 0,97 | 0,935 | 0,92 |
| 5 | 0,965 | 0,94 | 0,9 | 0,85 | 0,81 |
| 6 | 0,805 | 0,62 | 0,46 | - | - |
| 7 | 0,94 | 0,875 | 0,82 | 0,78 | 0,74 |
| 8 | 0,93 | 0,87 | 0,83 | - | - |
| 9 | 0,98 | 0,95 | 0,93 | 0,94 | 0,94 |
| 10 | 0,97 | 0,965 | 0,95 | 0,94 | 0,93 |

Die Daten in Tabelle 2 legen nahe, daß die Chemikalien der Beispiele 1, 4, 9 und 10 unter den Testbedingungen genügend stabil waren, da sie nach zwei Tagen Erwärmung mehr als 90% des aktiven Materials enthielten. Die Chemikalien der Beispiele 2, 5, 7 und 8 wurden abgebaut, aber nach einem Tag war immer noch mehr als 80% des ursprünglichen aktiven Stoffs vorhanden. Die Chemikalien der Beispiele 3 und 6 zeigten einen signifikanten Abbau.

BEISPIEL 12

Thermischer Abbau, Versuche bei 315°C und Luft mit 1010 Weichstahl

Das Verfahren des Beispiels 11 wurde wiederholt, außer daß der Muffelofen für diese Versuchsserie auf 315°C eingestellt wurde. Die Infrarotfrequenzen, die in Tabelle 1 aufgeführt sind, wurden ebenfalls für die Aufnahme dieser Daten freie sowie jene des Beispiels 13 verwendet, wobei die normalisierten Daten in Tabelle 3 bzw. 4 aufgeführt sind.

Tabelle 3

| Ergebnisse des Abbaus von verschiedenen Chemikalien bei 315°C unter Luft mit 1010 Weichstahl | | | | |
|---|---|---|---|---|
| Normalisiertes Verhältnis (aktiv/inaktiv) | | | | |
| Beispiel Nr. | nach 5 Std. | nach 10 Std. | nach 20 Std. | nach 30 Std. | nach 40 Std. |
| 1 | 0,8 | 0,67 | 0,47 | 0,34 | 0,225 |
| 2 | 0,86 | 0,765 | 0,635 | 0,54 | - |
| 3 | 0,505 | 0,35 | 0,235 | - | - |
| 4 | 0,975 | 0,955 | 0,905 | 0,86 | 0,83 |
| 5 | 0,905 | 0,825 | 0,75 | - | - |
| 6 | 0,68 | 0,555 | - | - | - |
| 7 | 0,89 | 0,82 | 0,755 | 0,67 | 0,57 |
| 8 | 0,78 | 0,61 | - | - | - |
| 9 | 0,95 | 0,925 | 0,88 | 0,83 | 0,78 |
| 10 | 0,93 | 0,91 | 0,88 | 0,82 | 0,745 |

Die obigen Ergebnisse zeigen, daß die Chemikalien der Beispiele 4, 9 und 10 gute thermische Stabilitäten haben, wobei mehr als 85% des ursprünglich aktiven Stoffes nach 24 Stunden unter den aufgeführten Bedingungen vorhanden ist. Die Chemikalien der Beispiele 2, 5 und 7 zeigen einen marginalen Abbau, während jede der Beispiele 1, 3, 6 und 8 einen signifikanten Abbau zeigen.

BEISPIEL 13

Thermische Abbauversuche bei 340°C unter Luft mit 1010 Weichstahl

Das Verfahren des Beispiels 11 wurde wiederholt, außer daß der Muffelofen für diese Serie auf 340°C eingestellt wurde. Die normalisierten Daten erscheinen in Tabelle 4.

Tabelle 4

| Ergebnisse des Abbaus von verschiedenen Chemikalien bei 340°C unter Luft mit 1010 Weichstahl | | | | |
|---|---|---|---|---|
| Normalisiertes Verhältnis (aktiv/inaktiv) | | | | |
| Beispiel Nr. | nach 1 Std. | nach 4 Std. | nach 7 Std. | nach 10 Std. | nach 20 Std. |
| 1 | 0,79 | 0,47 | 0,33 | - | - |
| 2 | 0,81 | 0,64 | 0,43 | - | - |
| 3 | 0,61 | 0,4 | 0,33 | 0,27 | - |
| 4 | 0,87 | 0,805 | 0,71 | 0,67 | 0,555 |
| 5 | 0,97 | 0,82 | 0,7 | 0,62 | 0,41 |
| 6 | 0,42 | - | - | - | - |
| 7 | 0,92 | 0,71 | 0,595 | 0,505 | - |
| 8 | 0,84 | 0,64 | - | - | - |
| 9 | 0,965 | 0,835 | 0,715 | 0,675 | - |
| 10 | 0,97 | 0,92 | 0,87 | 0,825 | 0,67 |

Die obigen Daten zeigen, daß die Chemikalien der Beispiele 4, 5, 9 und 10 unter den Reaktionsbedingungen stabiler sind als die anderen Stoffe. In diesen Fällen ist mehr als 50% des aktiven Stoffs nach 15 Stunden vorhanden.

BEISPIEL 14A

Thermische Abbauversuche einer 40%igen aktiven synthetischen aromatischen Lösung bei 340°C unter Luft mit 1010 Weichstahl

In 1 ml Ampullen wurden 0,25 g 40 Gew.-%iger Lösungen, die die zu untersuchende Chemikalie in einem synthetischen aromatischen Lösungsmittel (Alkylat 230, Monsanto) enthielten, und 4 cm 1010 Weichstahldraht der Stärke 22 gegeben. Die Ampullen wurden unter Luft verschlossen und in einen Muffelofen bei 340°C gegeben. Nach verschiedenen Zeiten wurden einige der Ampullen entfernt, gekühlt und geöffnet. Die Inhalte wurden durch Infrarotspektroskopie untersucht. Wie in Beispiel 11 wurden die normalisierten Verhältnisse der Absorptionen der aktiven und inaktiven Banden bestimmt. Die verwendeten Frequenzen der untersuchten Chemikalien sind in Tabelle 5 dargestellt, während in Tabelle 6 die normalisierten Daten des beschriebenen Abbaus aufgeführt sind.

Tabelle 5

| Infrarotfrequenzen für die untersuchten Chemikalien im Lösungsmittel | | |
|---|---|---|
| Beispiel der untersuchten Chemikalie | Aktive Frequenz ($cm^{-1}$) | Inaktive Frequenz ($cm^1$) |
| 1 | 1600 | 1460 |
| 2 | 1600 | 1460 |
| 4 | 1635 | 1460 |
| 5 | 1640 | 1460 |
| 6 | 1635 | 1460 |
| 9 | 1640 | 1460 |
| 10 | 1630 | 1455 |

Tabelle 6

| Thermische Abbauversuche von 40%igen aktiven Lösungen verschiedener Chemikalien bei 340°C unter Luft mit 1010 Weichstahl | | | | | | |
|---|---|---|---|---|---|---|
| Normalisiertes Verhältnis (aktiv/inaktiv) | | | | | | |
| Beispiel Nr. | nach 1 h | nach 2 h | nach 5 h | nach 10 h | nach 20 h | nach 50 h |
| 1 | 0,72 | 0,64 | 0,43 | 0,335 | 0,075 | - |
| 2 | 0,46 | 0,33 | 0,19 | 0,165 | 0,09 | - |
| 4 | 0,97 | 0,945 | 0,875 | 0,805 | 0,66 | 0,415 |
| 5 | 0,93 | 0,9 | 0,82 | 0,695 | 0,605 | 0,25 |
| 6 | 0,73 | 0,48 | 0,27 | 0,17 | 0,06 | - |
| 9 | 0,92 | 0,8 | 0,685 | 0,625 | 0,57 | 0,36 |
| 10 | 0,98 | 0,96 | 0,95 | 0,93 | 0,865 | 0,555 |

Diese Daten zeigen, daß die Chemikalien der Beispiele 4 und 10 unter diesen Bedingungen am stabilsten sind. Die in den Beispielen hergestellten Chemikalien, die nicht in Tabelle 5 und 6 aufgeführt sind, wurden nicht untersucht, da sie nicht in dem aromatischen Lösungsmittel löslich waren. Die in Tabellen 5 und 6 aufgeführten Chemikalien können in schwere aromatische Lösungsmittel ohne Colösungsmittel formuliert werden.

BEISPIEL 14B

Thermische Abbauversuche einer 40%igen aktiven synthetischen aromatischen Lösung bei 400°C unter Luft mit 1010 Weichstahl

In 1 ml Ampullen wurden 0,25 g 40 Gew.-%iger Lösungen, die die zu untersuchende Chemikalie in einem synthetischen aromatischen Lösungsmittel (Alkylat 230, Monsanto) enthielten, und 4 cm 1010 Weichstahldraht der Stärke 22 gegeben. Die Ampullen wurden anschließend unter Luft verschlossen und in einen Muffelofen bei 400°C gegeben. Nach verschiedenen Zeiten wurden einige der Ampullen entnommen, gekühlt und geöffnet. Die Inhalte wurden durch Infrarotspektroskopie analysiert. Wie in Beispiel 11 wurden die normalisierten Verhältnisse der Absorptionen der aktiven und inaktiven Banden bestimmt. Die in diesem Beispiel verwendeten Frequenzen sind jene, die in Tabelle 5 des Beispiels 14A gezeigt sind. Tabelle 6.5 zeigt die normalisierten Daten, welche den Abbau beschreiben.

Tabelle 6.5

| Thermische Abbauversuche von 40%igen aktiven Lösungen verschiedener Chemikalien bei 400°C unter Luft mit 1010 Weichstahl | | | | | | |
|---|---|---|---|---|---|---|
| Normalisiertes Verhältnis (aktiv/inaktiv) | | | | | | |
| Beispiel Nr. | nach 0,1 h | nach 0,2 h | nach 0,4 h | nach 0,6 h | nach 0,8 h | nach 1,0 h |
| 1 | 0,76 | 0,6 | 0,32 | 0,22 | 0,14 | 0,07 |
| 2 | 0,8 | 0,6 | 0,37 | 0,32 | 0,17 | 0,01 |
| 4 | 0,99 | 0,98 | 0,86 | 0,71 | 0,56 | 0,4 |
| 5 | 0,9 | 0,78 | 0,58 | 0,41 | 0,28 | 0,15 |
| 6 | 0,58 | 0,15 | 0,1 | 0,06 | 0,05 | 0,04 |
| 9 | 0,87 | 0,77 | 0,52 | 0,34 | 0,23 | 0,12 |
| 10 | 0,96 | 0,92 | 0,82 | 0,68 | 0,51 | 0,34 |

Diese Daten legen nahe, daß die Chemikalien 4 und 10 unter den Reaktionsbedingungen am stabilsten sind.

BEISPIEL 15

Bewertung verschiedener Chemikalien als Korrosionsinhibitoren gemäß rotierendem Radversuch mit Lauge nach ASTM und Rohöl bei 60°C für 24 Stunden mit 1010 Weichstahlprobestücken

Eine Variation des Versuchs nach NACE ID182, I. 54238, zur Korrosionsinhibierung wurde verwendet, um die Chemikalien des Beispiels 1 bis 10 zu bewerten. Sieben Unzen Glasflaschen wurden mit 50 ml Lauge (ASTM D-1141) und 50 ml entwässertem kalifornischen Rohöl (API Gewicht von 26,9°) und einer geeigneten Menge der Versuchschemikalie (1% in Xylol) gegeben. Die Lauge wurde vier Stunden mit einem Kohlendioxidgasstrom gereinigt. Nach Zugabe eines gereinigten, zuvor gewogenen 1010 Weichstahlprobestücks wurden die Flaschen unter einem Strom von Schwefelwasserstoffgas (Ersetzen des Stickstoffs über der Lösung), der vier Sekunden dauerte, verschlossen. Die Flaschen wurden auf einem rotierenden Rad für 24 Stunden bei einer konstanten Temperatur von 60°C befestigt. Danach wurden die Probestücke aus den Flaschen entnommen, gereinigt und nochmals gewogen. Unbehandelte blanke Probestücke wurden ebenfalls in jedem Durchgang des Radtests verwendet. Der Schutz in % wurde anschließend durch folgende Formel bestimmt:

$$\text{\%-Schutz} = (100\%)(\Delta M_{ungeschützt} - \Delta M_{geschütztes\ Probestück})/\Delta M_{ungeschützt}$$

$\Delta M_{ungeschützt}$ = Gewichtsverlust des ungeschützten Probestücks
$\Delta M_{geschütztes\ Probestück}$ = Gewichtverlust des Testprobestückes

Tabelle 7

| Schutz durch verschiedene Chemikalien in Prozent | | | | | |
| --- | --- | --- | --- | --- | --- |
| Schutz in Prozent | | | | | |
| Beispiel Nr. | bei 3 ppm | bei 10 ppm | bei 25 ppm | bei 50 ppm | bei 100 ppm |
| 1 | 70 | 76 | 79 | 83 | 85 |
| 2 | 55 | 62 | 71 | 74 | 82 |
| 3 | 56 | 63 | 74 | 78 | 81 |
| 4 | 57 | 63 | 67 | 77 | 84 |
| 5 | 47 | 53 | 61 | 73 | 84 |
| 6 | 46 | 46 | 46 | 49 | 51 |
| 7 | 46 | 48 | 53 | 57 | 59 |
| 8 | 49 | 55 | 58 | 61 | 62 |
| 9 | 52 | 61 | 68 | 72 | 83 |
| 10 | 49 | 50 | 59 | 68 | 75 |

Die Daten in Tabelle 7 zeigen, daß die Chemikalie des Beispiels 1 einen ausgezeichneten Schutz für Weichstahl bietet, wenn sie nicht einem Abbau durch hohe Temperaturen unterliegt. Ein guter Schutz wird ebenfalls durch die Chemikalien der Beispiele 2, 3, 4 und 9 zur Verfügung gestellt. Ein ausreichender Schutz wird durch die Chemikalien der Beispiele 5 und 10 bei Verwendung hoher Konzentration zur Verfügung gestellt. Dieses Testverfahren legt nahe, daß die Chemikalien der Beispiele 6, 7 und 8 nur einen geringen Schutz bieten.

BEISPIEL 16

Bewertung verschiedener Chemikalien nachdem diese einer Wärmebehandlung bei 315°C unterzogen wurden

Das Verfahren des Beispiels 15 wurde wiederholt, wobei die gleichen Chemikalien verwendet wurden, aber sie 24 Stunden einer Temperatur von 315° in verschlossenen Röhren ausgesetzt. Die Ergebnisse sind in Tabelle 8 dargestellt.

Tabelle 8

| Schutz in % für wärmebehandelte (315°C) Chemikalien | | | | | |
| --- | --- | --- | --- | --- | --- |
| Schutz in Prozent | | | | | |
| Beispiel Nr. | bei 3 ppm | bei 10 ppm | bei 25 ppm | bei bei 50 ppm 100 ppm | |
| 1 | 23 | 30 | 40 | 47 | 54 |
| 2 | 20 | 29 | 39 | 48 | 52 |
| 3 | 15 | 19 | 28 | 34 | 44 |
| 4 | 51 | 60 | 65 | 72 | 77 |
| 5 | 44 | 51 | 56 | 68 | 74 |
| 6 | 19 | 18 | 25 | 30 | 35 |
| 7 | 20 | 26 | 35 | 40 | 55 |
| 8 | 8 | 21 | 25 | 30 | 38 |
| 9 | 49 | 55 | 62 | 68 | 75 |
| 10 | 43 | 53 | 66 | 71 | 76 |

Die Daten in Tabelle 8 zeigen, daß die Chemikalien der Beispiele 4, 5, 9 und 10 einen guten Korrosionsschutz zur Verfügung stellen, nachdem sie 24 Stunden auf 315°C erwärmt wurden. Bei diesen Testbedingungen zeigen die anderen Chemikalien einen schlechten Schutz für Weichstahl.

BEISPIEL 17

Bewertung von verschiedenen Chemikalien, nachdem sie einer Wärmebehandlung bei 340°C unterzogen wurden.

Das Verfahren des Beispiels 15 wurde wiederholt, außer daß die zu untersuchenden Chemikalien auf 340°C für 24 Stunden in verschlossenen Röhren erwärmt wurden. Die Daten sind in Tabelle 9 dargestellt.

Tabelle 9

| Schutz in % für verschiedene wärmebehandelte (340°C) Chemikalien | | | | | |
|---|---|---|---|---|---|
| Schutz in Prozent | | | | | |
| Beispiel Nr. | bei 3 ppm | bei 10 ppm | bei 25 ppm | bei 50 ppm | bei 100 ppm |
| 1 | 6 | 15 | 19 | 20 | 22 |
| 2 | 6 | 15 | 12 | 13 | 20 |
| 3 | 5 | 13 | 17 | 20 | 23 |
| 4 | 41 | 46 | 49 | 58 | 61 |
| 5 | 29 | 35 | 40 | 42 | 48 |
| 6 | 4 | 8 | 8 | 6 | 6 |
| 7 | 26 | 27 | 33 | 34 | 35 |
| 8 | 5 | 8 | 12 | 11 | 14 |
| 9 | 30 | 32 | 38 | 38 | 46 |
| 10 | 39 | 50 | 56 | 58 | 64 |

Die Dazen in Tabelle 9 zeigen, daß die Chemikalien der Beispiele 4 und 10 einen guten Schutz bei mittleren und hohen Konzentrationen zur Verfügung stellen. Die Chemikalien der Beispiele 5, 7 und 9 zeigen einen ausreichenden Schutz, falls in hohen Konzentrationen verwendet werden, während die verbleibenden Chemikalien nur einen geringen Schutz unter diesen Versuchsbedingungen für 1010 Weichstahl zur Verfügung stellen.
Das aromatische Aminoamid des Beispiels 7 zeigte einen geringfügig geringeren Schutz als die cycloaliphatischen Aminoamide der Beispiele 5 und 9.
Die Korrosionsversuche zeigen, daß unverzweigte Aminoamide der allgemeinen Formel der vorliegenden Erfindung eine viel höhere korrosionsinhibierende Wirksamkeit haben, als Aminoamide mit einem aromatischen Ring im Molekül, wie das spezielle Diamin des Beispiels 3 und Diaminoamide mit einem Imidazolring.

BEISPIEL 18

Wertung verschiedener Chemikalien in einem verschlossenen Autoklav bei 320°C unter Druck

In ein 300 ml SS-316 Autoklav (Parr Model 4841) wurden 70 ml 6% ASTM-Lauge, 70 ml Rohöl mit einem API-Gewicht von 22,1° und ein magnetischer Rührstab gegeben. Gewogene und gereinigte Weichstahlprobestücke wurden an dem Deckel des Autoklavs befestigt, so daß, falls dieser verschlossen ist, die Probestücke teilweise in die gerührte Flüssigkeit eingetaucht sind. Wenn ein Versuchsinhibitor in einem Durchgang verwendet wurde, wurde er in die flüssige Mischung unter Verwendung einer Mikropipette injiziert, so daß die Konzentration des Inhibitors 100 ppm betrug. Sobald der Autoklav verschlossen war, wurde er auf 500 PSIG mit einer Gasmischung, die 98,8% Methan, 1,0% Kohlendioxid und 0,2% Schwefelwasserstoff enthielt, unter Druck gesetzt. Das System wurde extern auf 320°C erwärmt und 24 Stunden in diesem Zustand gehalten. Die sich hieraus ergebenden Drücke wurden für die 320°C-Bedingung aufgezeichnet. Danach wurde das System auf Raumtemperatur gekühlt und geöffnet. Die Probestücke wurden entnommen, gereinigt, gewogen und untersucht. Die Ergebnisse sind in Tabelle 10 dargestellt.

| Tabelle 10 | | | |
|---|---|---|---|
| Untersuchte Chemikalie | System-druck | %-Schutz | Kommentar |
| Beispiel 1 | 2250 -2440 PSIG | 11,4 | Verfärbung und Verschmutzung, allgemeine und Lochfraß-Korrosion beobachtet |
| Beispiel 4 | 2280 PSIG | 41,6 | allgemeine Korrosion beobachtet |
| Beispiel 5 | 2320 PSIG | 33,3 | schwache Verfärbung und allgemeine Korrosion beobachtet |
| Beispiel 10 | 2260 PSIG | 44,6 | allgemeine Korrosion beobachtet |

Diese Ergebnisse zeigen, daß die erfindungsgemäße Aminoamide eine gute Inhibierung bei hohen Temperaturen unter Druck ermöglichen, wohingegen die üblichen Imidazoline versagen.

**Patentansprüche**

1.  Zusammensetzung zur Inhibierung der Korrosion von Metalloberflächen, aufweisend neben üblichen Zusätzen 1 bis 99 Gew.-% der Zusammensetzung eines Aminoamids der allgemeinen Formel

$$R^1\text{-}CO\text{-}NR^2\text{-}Z\text{-}NR^3R^4 \hspace{4cm} (I)$$

worin Z ein divalenter aliphatischer Rest ist, der eine verzweigte Kohlenstoffkette hat oder ein divalentes alicyclisches Ringsystem einschließlich von Ringsystemen mit ein oder zwei gebundenen Alkylengruppen und/oder Alkylgruppen, wobei die Reste eine Kohlenstoffkette mit wenigstens drei Kohlenstoffatomen zwischen den gebundenen $NR^2$- und $NR^3R^4$-Gruppen aufweisen und worin das Strukturelement $-NR^2Z\text{-}NR^3R^4$ der Formel (I) kein Imidazolin oder Tetrahydropyrimidin Ringsystem bildet,
$R^2$, $R^3$, $R^4$ jeweils unabhängig voneinander Wasserstoff, einen aliphatischen, alicyclischen oder aromatischen Rest bedeuten,
$R^1$ ein monovalenter Monomer- oder Polymerrest ist, welcher einen oleophilen Anteil hat, der einen gesättigten oder olefinischen, linearen oder verzweigten Kohlenwasserstoffrest, einen alicyclischen oder aromatischen Rest aufweist,
oder eines Salzes davon.

2.  Zusammensetzung nach Anspruch 1, dadurch gekennzeichnet, daß die Verbindung der Formel (I) oder deren Salz in einem aliphatischen, aromatischen, alkylaromatischen oder cycloaliphatischen Kohlenwasserstoff oder in Mischungen daraus gelöst ist oder in Wasser oder einer wässrig-organischen Lösungsmittelmischung dispergiert ist.

3.  Zusammensetzung nach Anspruch 1 oder 2, dadurch gekennzeichnet, daß $R^1$ Alkyl, Alkylen oder Alkaldienyl mit 3 bis 30 Kohlenstoffatomen ist.

4.  Zusammensetzung nach Anspruch 1, 2 oder 3, dadurch gekennzeichnet, daß $R^1$ eine lineare Alkyl- oder Alkylengruppe mit 7 bis 22 Kohlenstoffatomen ist.

5.  Zusammensetzung nach einem der vorhergehenden Ansprüche, dadurch gekennzeichnet, daß $R^2$, $R^3$ und $R^4$ gleich oder verschieden Wasserstoff oder Alkyl mit 1 bis 5 Kohlenwasserstoffatomen sind.

6.  Zusammensetzung nach einem der vorhergehenden Ansprüche, dadurch gekennzeichnet, daß Z eine verzweigte Alkylengruppe mit 6 bis 10 Kohlenstoffatomen ist und 4 bis 6 Kohlenstoffatome zwischen den gebundenen Aminogruppen hat.

7.  Zusammensetzung nach einem der vorhergehenden Ansprüche 1 bis 5, dadurch gekennzeichnet, daß Z ein divalenter Kohlenwasserstoffrest mit 6 bis 12 Kohlenstoffatomen oder ein mono-, bi- oder tricyclisches fünf- und/oder sechsgliedriges Ringsystem ist.

8. Zusammensetzung nach einem der vorhergehenden Ansprüche 1 bis 5, dadurch gekennzeichnet, daß Z ein cycloaliphatisches Ringsystem ist.

9. Zusammensetzung nach Anspruch 1, dadurch gekennzeichnet, daß das Aminoamid ein Reaktionsprodukt einer höheren Fettsäure insbesondere von Talgölfettsäure mit Isophorondiamin, 2(3),5(6)-Diaminomethylnorbornylen, 1,8-Diamino-p-menthan oder 2,2,4-Trimethyl-1,6-diaminohexan ist.

10. Verwendung der Zusammensetzung gemäß den Ansprüchen 1 bis 9 in einer wirksamen Menge zur Inhibierung der Korrosion auf Metalloberflächen.

11. Verwendung nach Anspruch 10, dadurch gekennzeichnet, daß die Zusammensetzung als solche in Kontakt mit der Metalloberfläche gebracht wird.

12. Verwendung nach Anspruch 10, dadurch gekennzeichnet, daß die Zusammensetzung mit der Metalloberfläche in flüssiger Phase kontaktiert wird.

13. Verwendung nach einem der Ansprüche 10 bis 12, dadurch gekennzeichnet, daß die Metalloberfläche mit dem Korrosionsinhibitor bei Temperaturen von oberhalb 150°C kontaktiert wird.

14. Verwendung nach Anspruch 13, dadurch gekennzeichnet, daß der Kontakt unter einem Druck von oberhalb 9 MPa geschieht.

15. Verwendung nach einem oder mehreren der Ansprüche 10 bis 14, dadurch gekennzeichnet, daß Korrosion von Metalloberflächen von Ausrüstungsgegenständen aus Metall in Gas- oder Ölquellen, in heißen Tiefbohrungen nach Gas oder Öl, bei Raffinerievorgängen oder in geothermischen heißen Tiefbohrungen inhibiert wird und daß die Zusammensetzung als solche, als Lösung oder als Dispersion, in die Gas- oder Ölquellen injiziert wird, wobei die Injektion chargenweise oder kontinuierlich stattfindet.

16. Verwendung nach einem der Ansprüche 10 bis 15, dadurch gekennzeichnet, daß die zu schützende Metalloberfläche ausgewählt ist aus der Gruppe bestehend aus Weichstahl (unlegierter Stahl, C-Stahl), Schmiedeeisen, Gußeisen und Edelstählen.

17. Verwendung nach einem der Ansprüche 10 bis 16, dadurch gekennzeichnet, daß die Zusammensetzung mit der Metalloberfläche in Kontakt gebracht wird, indem man die Metalloberfläche mit der Zusammensetzung als solcher oder in flüssiger Phase einreibt oder besprüht oder die Metalloberfläche in die Zusammensetzung als solche oder in flüssiger Phase taucht.

18. Verwendung nach einem der Ansprüche 10 bis 17, dadurch gekennzeichnet, daß die Metalloberfläche gegen Korrosion geschützt wird, die durch Säuren, $CO_2$, $H_2S$, Laugen (Salzlaugen), Sauerstoff oder Luft oder Mischungen daraus verursacht wird.

19. Verwendung nach einem der Ansprüche 10 bis 18, dadurch gekennzeichnet, daß die Korrosionsinhibierung bei Temperaturen im Bereich von 190 bis 450°C erreicht wird.

20. Verwendung nach einem der Ansprüche 10 bis 19, dadurch gekennzeichnet, daß die Zusammensetzung in einem fließenden Fluidsystem in einer Menge von etwa 10 bis 100 Teilen Inhibitor pro eine Million Teile des Fluids eingesetzt wird.

21. Verwendung nach einem der Ansprüche 10 bis 19, dadurch gekennzeichnet, daß die Zusammensetzung in einem statischen Fluidsystem in einer Menge von etwa 1 bis 10000 Teilen Inhibitor pro eine Million Teile des Fluids eingesetzt wird.

22. Verwendung nach einem der Ansprüche 10 bis 19, dadurch gekennzeichnet, daß die Zusammensetzung in einen Kunststoff eingeschlossen ist, um die Korrosion einer Metalloberfläche zu verhindern, die durch den Kunststoff in fester oder geschmolzener Form berührt wird.

23. Aminoamid der allgemeinen Formel

$$R^1\text{-CO-NR}^2\text{-Z-NR}^3R^4 \qquad\qquad (I)$$

gemäß den Ansprüchen 1 bis 9, wobei das Aminoamid das Reaktionsprodukt eines Amins der Formel

$$R^2\text{-NH-Z-NR}^3R^4$$

mit $R^1$-COOH oder einem reaktiven Derivat davon ist, oder ein Salz davon, mit der Bedingung, daß Fettsäureamide des Isophorondiamins und von Diamidotriaminen basierend auf Glutarsäure ausgeschlossen sind.

| | **Europäisches Patentamt** | **EUROPÄISCHER RECHERCHENBERICHT** | | **Nummer der Anmeldung** **EP 98 11 8262** |
|---|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US 3 167 554 A (ERNST R.) 26. Januar 1965<br><br>* Spalte 1, Zeile 19-40 *<br>* Spalte 10, Zeile 37-49 *<br>--- | 1-3,5,7, 8,10-12, 15-18,23 | C23F11/14 E21B41/02 C07C233/35 |
| X | US 3 265 512 A (DICKSON W. J.) 9. August 1966<br><br>* Spalte 2, Zeile 39-41; Anspruch 1 *<br>--- | 1-5, 10-12, 15-18,23 | |
| X | EP 0 294 641 A (BAYER AG) 14. Dezember 1988<br><br>* Seite 8, Zeile 14-57; Anspruch 3 *<br>--- | 1,2,5, 7-12, 15-18, 20,21,23 | |
| X | US 2 995 603 A (HUTCHISON C. B.) 8. August 1961<br><br>* Spalte 3, Zeile 55; Anspruch 1 *<br>* Spalte 2, Zeile 36-56 *<br>--- | 1,2,5, 10-12, 15-18,23 | **RECHERCHIERTE SACHGEBIETE (Int.Cl.6)** |
| X | GB 1 109 579 A (FARBWERKE HOECHST AG)<br><br>* Seite 1, Zeile 10-48 *<br>--- | 1-5, 10-12, 15-18,23 | C23F E21B C07C |
| X | PATENT ABSTRACTS OF JAPAN vol. 006, no. 094 (C-105), 2. Juni 1982 & JP 57 026174 A (TAKEDA CHEM IND LTD), 12. Februar 1982 * Zusammenfassung * --- | 1-3,5,7, 8,10-12, 15-18,23 | |

-/--

Der vorliegende Recherchenbericht wurde für alle Patentansprüche erstellt

| Recherchenort | Abschlußdatum der Recherche | Prüfer |
|---|---|---|
| DEN HAAG | 11. Januar 1999 | Torfs, F |

Europäisches
Patentamt

**EUROPÄISCHER RECHERCHENBERICHT**

Nummer der Anmeldung

EP 98 11 8262

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | FR 2 304 688 A (ASAHI CHEMICAL IND) 15. Oktober 1976<br><br>* Seite 16, Zeile 2-4; Anspruch 1; Beispiele 3,4 *<br>--- | 1-7, 10-12, 15-18,23 | |
| X | GB 1 062 359 A (ARMOUR AND CO) 22. März 1967<br><br>* Ansprüche 1,2,6 *<br>--- | 1-5, 10-12, 15-18,23 | |
| X,D | DATABASE WPI Section Ch, Week 9236 Derwent Publications Ltd., London, GB; Class E19, AN 92-295558 XP002089572 & JP 04 202396 A (KYOEISHA OIL CHEM IND CO), 23. Juli 1992 * Zusammenfassung *<br>--- | 1-5,7-9 | |
| X | GB 2 305 670 A (COATES BROTHERS PLC) 16. April 1997 * Ansprüche 7,9; Beispiel 3 *<br>--- | 1,3-9,23 | RECHERCHIERTE SACHGEBIETE (Int.Cl.6) |
| X | EP 0 211 978 A (NICHOLS GUS) 4. März 1987 * Anspruch 7 *<br>--- | 1,3-9,23 | |
| X | US 4 675 374 A (NICHOLS GUS) 23. Juni 1987 * Anspruch 8 *<br>--- | 1,3-9,23 | |
| X | US 5 039 721 A (SCHWERZEL THOMAS ET AL) 13. August 1991 * Spalte 3, Zeile 7-24; Anspruch 1 *<br>--- | 1,3-9,23 | |
| A | BE 689 748 A (BASF) 16. Mai 1967 * Anspruch 1 *<br>--- | 22 | |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 11. Januar 1999 | Torfs, F |

EP 0 906 969 A1

**Europäisches Patentamt**

**EUROPÄISCHER RECHERCHENBERICHT**

Nummer der Anmeldung

EP 98 11 8262

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP 0 060 456 A (BASF AG) 22. September 1982 --- | | |
| A | EP 0 256 802 A (BETZ EUROP INC) 24. Februar 1988 --- | | |
| A,D | US 5 391 826 A (SPERANZA GEORGE P ET AL) 21. Februar 1995 ----- | | |

| | | | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| DEN HAAG | 11. Januar 1999 | Torfs, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................................

& : Mitglied der gleichen Patentfamilie, übereinstimmendes Dokument

EPO FORM 1503 03.82 (P04C03)

23

**ANHANG ZUM EUROPÄISCHEN RECHERCHENBERICHT**
**ÜBER DIE EUROPÄISCHE PATENTANMELDUNG NR.**                    EP 98 11 8262

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-01-1999

| Im Recherchenbericht angeführtes Patentdokument | Datum der Veröffentlichung | Mitglied(er) der Patentfamilie | Datum der Veröffentlichung |
|---|---|---|---|
| US 3167554 A | 26-01-1965 | KEINE | |
| US 3265512 A | 09-08-1966 | US 3200106 A<br>US 3259578 A<br>US 3259586 A<br>US 3262791 A | 10-08-1965<br>05-07-1966<br>05-07-1966<br>26-07-1966 |
| EP 0294641 A | 14-12-1988 | DE 3719101 A<br>DK 304588 A<br>US 4902830 A | 15-12-1988<br>07-12-1988<br>20-02-1990 |
| US 2995603 A | 08-08-1961 | KEINE | |
| GB 1109579 A | | DE 1545298 A<br>FR 1493820 A<br>NL 6612075 A<br>NL 6612093 A<br>SE 316327 B | 31-07-1969<br>08-12-1967<br>01-03-1967<br>01-03-1967<br>20-10-1969 |
| FR 2304688 A | 15-10-1976 | JP 958836 C<br>JP 51108644 A<br>JP 53043377 B<br>DE 2611187 A<br>GB 1499275 A<br>US 4028055 A<br>US 4073618 A | 14-06-1979<br>27-09-1976<br>18-11-1978<br>30-09-1976<br>25-01-1978<br>07-06-1977<br>14-02-1978 |
| GB 1062359 A | | KEINE | |
| GB 2305670 A | 16-04-1997 | AU 7137496 A<br>EP 0852608 A<br>WO 9712003 A | 17-04-1997<br>15-07-1998<br>03-04-1997 |
| EP 0211978 A | 04-03-1987 | AT 108810 T<br>DE 3587884 D<br>DE 3587884 T<br>US 4547562 A | 15-08-1994<br>25-08-1994<br>27-10-1994<br>15-10-1985 |
| US 4675374 A | 23-06-1987 | US 4547562 A<br>CA 1262003 A<br>JP 62062810 A<br>US 4742147 A | 15-10-1985<br>26-09-1989<br>19-03-1987<br>03-05-1988 |
| US 5039721 A | 13-08-1991 | DE 3825562 A<br>EP 0352671 A<br>JP 2069578 A | 01-02-1990<br>31-01-1990<br>08-03-1990 |

EPO FORM P0461

Für nähere Einzelheiten zu diesem Anhang : siehe Amtsblatt des Europäischen Patentamts, Nr.12/82

**EP 0 906 969 A1**

**ANHANG ZUM EUROPÄISCHEN RECHERCHENBERICHT**
**ÜBER DIE EUROPÄISCHE PATENTANMELDUNG NR.**     EP 98 11 8262

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-01-1999

| Im Recherchenbericht angeführtes Patentdokument | | Datum der Veröffentlichung | Mitglied(er) der Patentfamilie | | Datum der Veröffentlichung |
|---|---|---|---|---|---|
| US 5039721 | A | | US | 5130350 A | 14-07-1992 |
| BE 689748 | A | 16-05-1967 | BE | 689340 A | 08-05-1967 |
| | | | CH | 460739 A | |
| | | | DE | 1256887 B | |
| | | | DE | 1518611 A | 25-05-1972 |
| | | | FR | 1499785 A | 15-01-1968 |
| | | | FR | 1501268 A | 05-02-1968 |
| | | | NL | 6616027 A | 18-05-1967 |
| | | | SE | 326174 B | 20-07-1970 |
| EP 0060456 | A | 22-09-1982 | DE | 3109826 A | 23-09-1982 |
| | | | CA | 1178578 A | 27-11-1984 |
| EP 0256802 | A | 24-02-1988 | US | 5019341 A | 28-05-1991 |
| | | | CA | 1314704 A | 23-03-1993 |
| US 5391826 | A | 21-02-1995 | KEINE | | |

EPO FORM P0461

Für nähere Einzelheiten zu diesem Anhang : siehe Amtsblatt des Europäischen Patentamts, Nr. 12/82